# FASCICULE DE BREVET EUROPEEN

(11) **EP 2 712 549 B1**
(45) Date de publication et mention de la délivrance du brevet: **11.03.2015**
(21) Numéro de dépôt: 13185652.8
(22) Date de dépôt: 24.09.2013
(51) Int. Cl.: A61B 5/11, A61N 1/368, A61B 8/08, A61B 5/02, A61N 1/365, A61B 5/00, A61B 5/0215, A61B 5/0452, A61B 5/053

(54) **Dispositif d'évaluation de la désynchronisation ventriculaire temporelle**
Vorrichtung zur Bewertung der zeitlichen ventrikulären Desynchronisation
Device for assessing temporal ventricular dyssynchrony

(30) Priorité: 01.10.2012 FR 1259252
(43) Date de publication de la demande: 02.04.2014
(73) Titulaire: Sorin CRM SAS, 92140 Clamart Cedex (FR)
(72) Inventeur: Renesto, Fabrizio, 10013 Borgofranco d'Ivrea (TO) (IT); Henry, Christine, 75014 Paris (FR)
(74) Mandataire: Dupuis-Latour, Dominique

(56) Documents cités:
- EP-A1- 1 350 539
- EP-A1- 2 495 013
- WO-A1-95/27531
- WO-A1-99/30777
- WO-A1-99/58191
- US-A1- 2005 027 320
- US-B1- 7 212 861

## Description

L'invention concerne le diagnostic et le traitement de la désynchronisation ventriculaire chez un patient.

La désynchronisation ventriculaire est une pathologie qui se manifeste sous deux formes (pouvant se présenter isolément ou conjointement) : la désynchronisation spatiale et la désynchronisation temporelle.

Dans la désynchronisation dite "spatiale", un trouble de la conduction cardiaque introduit un retard de contraction d'un ventricule par rapport à l'autre ou à l'intérieur du ventricule gauche, ce qui conduit à une dégradation de l'état hémodynamique du patient.

Le US 2005/0027320 A1 décrit un tel dispositif de détection de la désynchronisation entre les deux ventricules (désynchronisation "spatiale"), permettant d'évaluer et d'appliquer une thérapie de stimulation appropriée notamment par optimisation du délai interventriculaire (DVV).

La désynchronisation dite "temporelle", en revanche, ne concerne qu'un seul ventricule, le ventricule gauche. Elle est caractérisée par un retard de contraction de tout ou partie du ventricule gauche par rapport à la fermeture de la valve aortique. Certains des segments de la paroi ventriculaire se trouvent encore dans un état de contraction pendant la phase diastolique,c'est-à-dire après la fin de l'éjection du sang dans l'aorte (cet instant correspondant à la fermeture de la valve aortique) voire, dans certains cas extrêmes, après l'ouverture de la valve mitrale, c'ést-à-dire au début de la phase de remplissage passif du ventricule par le sang refoulé par l'oreillette.

La désynchronisation ventriculaire temporelle est ainsi caractérisée par la présence d'une contraction diastolique, donc à contretemps, de certains segments du ventricule gauche, avec un effet antagoniste sur l'activité hémodynamique et une diminution de la fraction d'éjection, considérée comme le paramètre hémodynamique de référence.

Ces désynchronisations ventriculaires peuvent être traitée par une technique dite "CRT" (*Cardiac Resynchronization Therapy*) ou "BVP" *(BiVentricular Pacing*), consistant à implanter chez le patient un appareil et des sondes munies d'électrodes permettant de stimuler l'un et l'autre ventricule en divers sites. Le dispositif applique entre les instants respectifs de stimulation des ventricules gauche et droit un délai dit "délai interventriculaire" (DW ou WD) variable, ajusté de manière à resynchroniser la contraction des ventricules avec optimisation fine de l'état hémodynamique du patient. Le DVV peut être nul, positif (le ventricule gauche étant stimulé après le ventricule droit), ou négatif (le ventricule droit étant stimulé après le ventricule gauche).

On connait diverses techniques d'évaluation globale de la fonction hémodynamique chez un patient.

Par exemple, le EP 0 108 446 A1 (ELA Medical) évalue cette fonction à partir d'une mesure de bioimpédance intracardiaque, qui est un paramètre représentatif du débit cardiaque et donc de la fraction d'éjection. Un autre exemple en est donné par le EP 2 092 885 A1 (ELA Medical), qui extrait d'un signal d'accélération endocardiaque et combine entre eux divers indicateurs représentatifs de l'état hémodynamique du patient.

Ces dispositifs opèrent toutefois un diagnostic non spécifique de la désynchronisation ventriculaire, sans distinction entre désynchronisation temporelle et désynchronisation spatiale.

Le WO 95/27531 A1 décrit un autre dispositif encore, muni notamment d'un ou plusieurs capteurs de vitesse ou d'accélération mesurant le déplacement de la paroi cardiaque afin de détecter la présence, ou non, de contractions du myocarde. En l'absence d'activité mécanique du coeur, le dispositif délivrera une thérapie antibradycardique appropriée (stimulation conventionnelle par exemple de type DDD ou DDI), en revanche en présence d'une activité mécanique la thérapie sera inhibée de manière à ne pas venir interférer avec les contractions spontanées du myocarde. Le capteur peut notamment être un capteur d'accélération endocardiaque (EA) dont le signal reflète, sous forme de deux pics, les deux bruits du coeur qui peuvent être perçus à la fermeture de la valve mitrale et à la fermeture de la valve aortique. La mesure de la hauteur de l'un ou l'autre pic du signal EA donne ainsi une indication de la présence, ou non, d'une activité mécanique du coeur.

Les US 7 212 861 B1 et EP 2 495 013 A1 sont des documents décrivant diverses techniques de recherche d'une configuration de stimulation optimale basées sur la détection de l'activité mécanique du ventricule. Mais, pas plus que le document précédent, ils ne considèrent le diagnostic ni le traitement spécifique d'une éventuelle désynchronisation ventriculaire.

Le problème de l'invention est de pouvoir disposer d'un moyen spécifique de diagnostiquer la désynchronisation temporelle du ventricule, indépendamment de toute considération liée à une éventuelle désynchronisation spatiale (qui peut cependant à titre subsidiaire faire l'objet d'une thérapie complémentaire).

En particulier, si l'on peut discerner entre désynchronisation temporelle et désynchronisation spatiale, on peut appliquer des thérapies différenciées selon le type de trouble. En effet, alors que pour remédier à une désynchronisation spatiale il suffit généralement (comme dans le cas du US 2005/0027320 A1 précité) d'appliquer une stimulation biventriculaire avec DVV, le traitement de la désynchronisation temporelle - qui ne concerne qu'un seul ventricule -, relève d'un ajustement beaucoup plus fin de divers paramètres de stimulation, pouvant inclure notamment le délai atrioventriculaire (DAV ou AVD) et/ou une combinaison des DAV et DVV.

La détection spécifique d'une désynchronisation temporelle pourra ainsi permettre de remédier à un trouble non traité, ou insuffisamment traité, par les thérapies CRT conventionnelles.

À cet effet, l'invention propose un dispositif d'évaluation et de thérapie de la désynchronisation ventriculaire temporelle chez un patient.

Ce dispositif est d'un type général connu, notamment d'après le WO 95/27531 A1 précité, comprenant :
- un capteur hémodynamique apte à délivrer un signal représentatif d'un flux sanguin dans les cavités gauches du myocarde ;
- au moins un capteur de mouvement apte à délivrer un signal représentatif d'un déplacement des parois du ventricule gauche du myocarde ;
- des premiers moyens d'analyse, aptes à déterminer un instant de fermeture de la valve aortique à partir du signal du capteur hémodynamique ;
- des seconds moyens d'analyse, aptes à détecter et évaluer un pic de contraction du ventricule gauche à partir du (des) signal(ux) du(des) capteur(s) de mouvement ; et
- des moyens de stimulation bi-, tri- ou multiventriculaire, aptes à délivrer des impulsions de stimulation destinées à être appliquées à des électrodes implantées respectivement en au moins un site de stimulation ventriculaire droit et au moins un site de stimulation ventriculaire gauche selon une configuration de stimulation courante avec application d'au moins un délai interventriculaire DVV et/ou atrioventriculaire DAV modifiables.

De façon caractéristique de l'invention, les seconds moyens d'analyse sont aptes à déterminer un instant dudit pic de contraction du ventricule gauche, et le dispositif comprend en outre des troisièmes moyens d'analyse, aptes à mesurer un écart temporel entre l'instant du pic de contraction du ventricule gauche et l'instant de fermeture de la valve aortique. Il comprend également des moyens aptes, si les troisièmes moyens d'analyse déterminent que l'instant du pic de contraction du ventricule gauche est postérieur à l'instant de fermeture de la valve aortique, à modifier le(s) DVV et/ou DAV de la configuration de stimulation courante dans un sens réduisant et annulant le retard du pic de contraction du ventricule gauche après fermeture de la valve aortique.

Le capteur hémodynamique peut notamment être un capteur du groupe comprenant : capteur implantable ou externe d'accélération endocardiaque ; capteur implantable de bioimpédance ; capteur implantable ou externe de détection de l'onde T ; capteur implantable de pression véntriculaire ; et les combinaisons de tels capteurs.

Le capteur de mouvement peut notamment être un capteur du groupe comprenant : capteur de mouvement implantable en un site endocavitaire, épicardique ou endocoronarien ; capteur externe de mouvement ; capteur de recueil d'un signal de tomographie de champ ; et les combinaisons de tels capteurs.

Dans une forme de réalisation préférentielle, le capteur hémodynamique est un capteur implantable apte à délivrer un signal d'accélération endocardiaque EA, et les premiers moyens d'analyse sont des moyens aptes à isoler dans le signal EA une composante EA2 correspondant au second pic d'accélération endocardiaque associé à la relaxation ventriculaire isovolumique sur un cycle cardiaque compris entre deux évènements ventriculaires successifs, et déterminer comme instant de fermeture de la valve aortique l'instant d'apparition du début de la composante EA2, notamment un instant de franchissement d'un seuil d'enveloppe d'énergie de la composante EA2.

D'autre part, le dispositif de l'invention peut être utilisé pour diagnostiquer non seulement la désynchronisation ventriculaire temporelle, mais également la désynchronisation ventriculaire spatiale.

Il comprend alors au moins deux capteurs de mouvement, aptes à délivrer des signaux représentatifs des déplacements respectifs des parois du ventricule gauche et droit du myocarde. Les seconds moyens d'analyse sont aptes à déterminer les instants des pics de contraction respectifs des ventricules gauche et droit, et il est en outre prévu des moyens d'évaluation de la désynchronisation ventriculaire spatiale chez le patient, aptes à déterminer un défaut de concomitance des pics de contraction respectifs des ventricules gauche et droit.

Pour traiter la désynchronisation spatiale ainsi diagnostiquée, le dispositif comprend des moyens de stimulation bi-, tri- ou multiventriculaire, aptes à délivrer des impulsions de stimulation destinées à être appliquées à des électrodes implantées respectivement en au moins un site de stimulation ventriculaire droit et au moins un site de stimulation ventriculaire gauche selon une configuration de stimulation courante avec application d'au moins un délai interventriculaire DVV et/ou atrioventriculaire DAV modifiables. Si les moyens d'évaluation de la désynchronisation temporelle déterminent que les instants des pics de contraction des ventricules gauche et droit sont tous deux postérieurs à l'instant de fermeture de la valve aortique, il est prévu de modifier le(s) DVV et/ou DAV de la configuration de stimulation courante dans un sens qui i) prioritairement, réduite et annule le retard de ces deux pics de contraction après fermeture de la valve aortique et ii) subsidiairement, réduit l'écart temporel entre ces deux pics de contraction.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre de façon simplifiée les cavités du coeur et les valves qui leur sont associées.
La Figure 2 illustre un coeur appareillé avec diverses sondes pourvues de capteurs hémodynamiques et de mouvement.
La Figure 3 est une série de quatre chronogrammes illustrant différents signaux caractéristiques qu'il est possible de recueillir au cours d'un cycle cardiaque.
La Figure 4 montre de façon plus détaillée l'allure du signal d'accélération endocardiaque au cours d'un cycle donné, avec les différents marqueurs temporels utilisés par l'invention.
La Figure 5 est une représentation sous forme de diagramme échocardiographique d'une situation typique de désynchronisation temporelle du ventricule gauche.
La Figure 6 est une série de quatre chronogrammes illustrant une situation typique de double désynchronisation, temporelle et spatiale, des ventricules.
La Figure 7 est un organigramme explicitant les diverses étapes successives du procédé selon l'invention de diagnostic et de traitement de la désynchronisation ventriculaire temporelle.

On va maintenant décrire à titre d'exemple illustratif un mode de réalisation de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition de signaux fournis notamment par des sondes endocavitaires, épicardiques et/ou endocoronariennes pourvues de capteurs appropriés.

L'invention peut notamment être appliquée aux dispositifs implantables tels que ceux des familles *Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

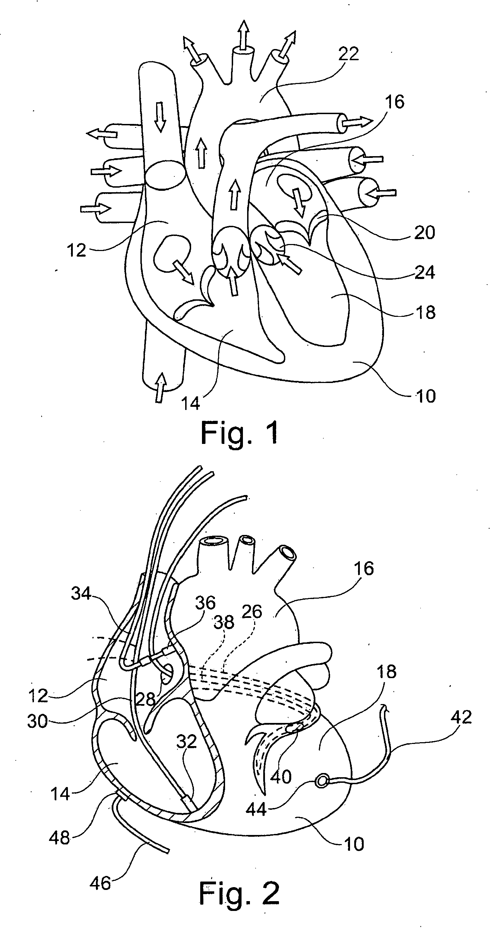

La Figure 1 illustre les cavités et les valves du coeur 10. On a représenté l'oreillette droite 12, le ventricule droit 14, l'oreillette gauche 16 et le ventricule gauche 18. La valve mitrale 20 (valve atrioventriculaire) est située entre l'oreillette gauche 16 et le ventricule gauche 18, et la valve aortique 24 (valve semilunaire) est située entre le ventricule gauche 18 et l'artère aorte 22.

La mise en oeuvre de l'invention nécessite de pouvoir suivre les variations du flux sanguin dans les cavités gauches et les mouvements de la paroi du ventricule gauche ainsi que, subsidiairement, les mouvements de la paroi du ventricule droit.

Le coeur 10 peut être appareillé à cet effet de diverses sondes pourvues de capteurs appropriés, comme illustré Figure 2.

Ces sondes peuvent être des sondes endocavitaires introduites dans l'oreillette droite 12 et/ou dans le ventricule droit 14, des sondes épicardiques fixées sur la paroi externe du myocarde, ou encore des sondes endocoronariennes dont les parties actives sont disposées vis-à-vis des cavités gauches, ces sondes étant introduites dans une veine 26 du réseau coronaire veineux via le sinus coronaire 28 débouchant dans l'oreillette droite 12.

La mise en oeuvre de l'invention nécessite en premier lieu un capteur hémodynamique délivrant un signal représentatif des variations du flux sanguin, plus particulièrement aux fins de détection de l'instant de fermeture de la valve aortique.

Un tel capteur peut être par exemple disposé sur une sonde endocavitaire aboutissant au fond du ventricule et pourvue à cet endroit d'un capteur 32 de détection de l'accélération endocardiaque (EA). Il peut s'agir également d'une sonde auriculaire 34 pourvue à son extrémité disposée contre la paroi de l'oreillette droite d'un capteur 36, également d'accélération endocardiaque. Un capteur EA est par exemple constitué à partir d'un accéléromètre intégré dans une tête de sonde endocavitaire, comme décrit dans le EP 0 515 319 A1 (SORIN Biomedica Cardio SpA).

De façon générale, le capteur hémodynamique peut être, outre un capteur EA intégré à une sonde endocavitaire, un capteur EA externe placé sur le thorax du patient, ou bien un capteur implantable de bioimpédance intracardiaque, un capteur implantable ou externe de détection de l'onde T du signal électrocardiographique, ou encore un capteur implantable de pression ventriculaire.

La mise en oeuvre de l'invention nécessite en second lieu un capteur de mouvement, apte à délivrer un signal représentatif d'un déplacement des parois du ventricule gauche 18.

Ce capteur de mouvement peut par exemple être placé sur une sonde endocoronarienne 38, en un ou plusieurs emplacements 40 disposés contre la paroi du ventricule gauche. En variante ou en complément, il est possible d'utiliser une sonde épicardique 42 pourvue d'un capteur d'extrémité 44 placé contre la paroi du ventricule gauche 18.

De façon subsidiaire, si l'on souhaite évaluer également le mouvement du ventricule droit, il est possible de prévoir une sonde appropriée telle que la sonde épicardique 46 pourvue d'un capteur d'extrémité 48 fixé sur une paroi externe du ventricule droit 14.

Il est possible d'utiliser plusieurs capteurs de mouvements placés en différents endroits de la paroi du ventricule gauche de manière à analyser plus précisément les mouvements des différents segments de cette paroi ventriculaire gauche. Ces divers capteurs peuvent être placés sur une même sondé, par exemple la sonde endocoronarienne 38, ou bien être distincts, comme dans le cas du capteur de la sonde épicardique 42.

D'autres techniques peuvent être utilisées pour obtenir une représentation du déplacement des parois du ventricule gauche, par exemple une technique d'analyse électrotomographique telle que celle décrite dans le US 2008/0183072 A1.

L'idée de base de l'invention consiste à obtenir, à partir du signal représentatif du flux sanguin dans les cavités gauches délivré par le capteur hémodynamique, une indication de l'instant de fermeture de la valve aortique, instant qui sera considéré comme un marqueur de référence pour le diagnostic de la désynchronisation temporelle. Le rapprochement de ce marqueur avec le signal délivré par le capteur de mouvement du ventricule gauche permettra de déterminer s'il existe ou non un état de contraction du ventricule gauche postérieur à ce marqueur temporel : dans l'affirmative, on considèrera qu'il y a présence d'une désynchronisation ventriculaire temporelle, et des mesures appropriées pourront être prises suite à ce diagnostic pour tenter de réduire ou faire disparaitre ce phénomène pathologique.

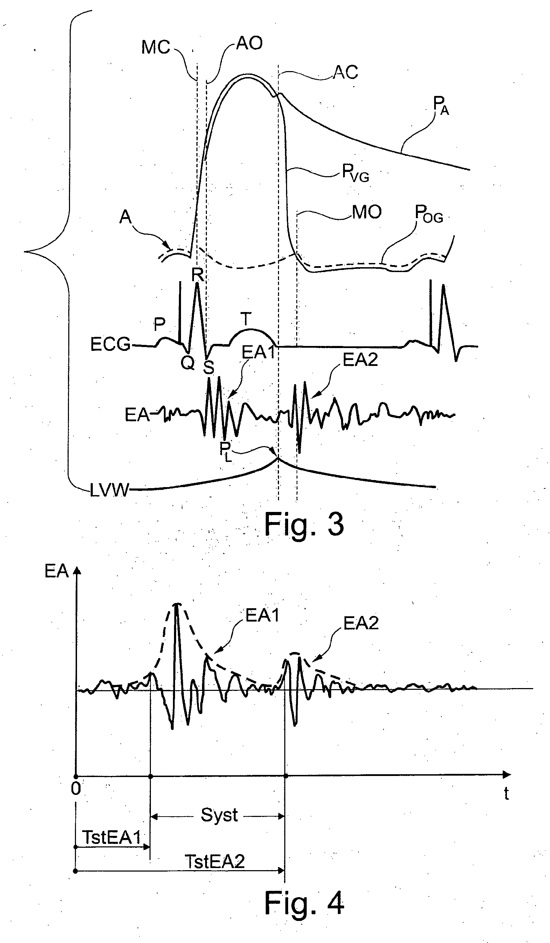

On va maintenant exposer, en référence aux Figures 3 et 4, la manière dont il est possible de déterminer ce marqueur temporel de référence (instant de fermeture de la valve aortique).

La Figure 3 illustre les différents signaux caractérisant l'activité du coeur au cours d'un cycle cardiaque avec :
- le profil des pressions intracardiaques P_{A}, P_{VG} et P_{OG} : la caractéristique P_{A} illustre les variations de la pression aortique, P_{VG} celles du ventricule gauche et P_{OG} celles de l'oreillette gauche. Ces variations passent par les différentes phases suivantes : A contraction de l'oreillette gauche, MC fermeture de la valve mitrale, AO ouverture de la valve aortique, AC fermeture de la valve aortique, MO ouverture de la valve mitrale ;
- un relevé d'électrocardiogramme de surface ECG, avec successivement : l'onde P correspondant à la dépolarisation des oreillettes, le complexe QRS correspondant à la dépolarisation des ventricules, et l'onde T de repolarisation ventriculaire ;
- les variations du signal EA d'accélération endocardiaque recueilli, qui forme deux composantes principales au cours d'un cycle cardiaque donné, correspondant aux deux bruits majeurs du coeur (sons S1 et S2 du phonocardiogramme) qu'il est possible de reconnaitre dans chaque cycle cardiaque; et
- les variations du signal LVW du capteur de mouvement, représentatif des déplacements de la paroi du ventricule gauche. Ce signal présente un pic P_{L} marquant la fin de la contraction de tous les segments du ventricule gauche.

Sur la Figure 4, on a illustré plus précisément les variations du signal EA au cours d'un cycle cardiaque, montrant :
- la composante EA1, qui débute à la suite du complexe QRS et qui est engendrée par une combinaison de la fermeture des valves atrioventriculaires (valve mitrale et valve tricuspide), de l'ouverture des valves semilunaires (valve aortique et valve pulmonaire), et de la contraction du ventricule gauche ; et
- la composante EA2, qui survient pendant la phase de relaxation ventriculaire isovolumique, accompagne la fin de la systole ventriculaire et est principalement produite pa la fermeture des valves aortique et pulmonaire.

Pour la mise en oeuvre de l'invention, il convient d'extraire du signal EA, et plus précisément de la composante EA2, un marqueur temporel caractéristique corrélé à la fermeture de la valve aortique (AC sur la Figure 3). L'analyse du signal EA est déterminée de préférence avec moyennage sur plusieurs cycles, typiquement trois à cinq cycles, en appliquant une technique telle que celle exposée dans le EP 2 092 885 A1 (ELA Medical), technique qui permet notamment d'éliminer les variations cycle-à-cycle en recalant dans le temps les composantes successives avant de les moyenner.

Essentiellement, cette technique consiste à effectuer un prétraitement du signal EA recueilli en continu, avec :
- découpage du signal EA en sous-signaux correspondant chacun à la durée d'un cycle cardiaque et repérés par des marqueurs de début de cycle (origine des temps) permettant de réaliser ce découpage. Les marqueurs temporels de début de cycle peuvent être fournis par le dispositif implanté qui, selon le mode de fonctionnement, garde en mémoire les instants de la stimulation V ou bien les instants de détection de l'onde R ;
- segmentation de chacun de ces sous-signaux de manière à individualiser les composantes EA1 et EA2 dans une fenêtre temporelle donnée ;
- pour la composante courante EA1 (ou EA2) ainsi isolée sur un cycle, recherche d'un pic d'intercorrélation par rapport aux composantes EA1 (ou EA2) des autres cycles recueillis ;
- calcul d'un décalage temporel correspondant ; et
- application du décalage temporel ainsi calculé à la composante courante, de manière à aligner celle-ci par rapport aux autres.

Les traitements d'analyse du signal EA pourront être ensuite exécutés sur les composantes EA1 et EA2 successives, avec élimination du biais de la variabilité cycle-à-cycle grâce à ce prétraitement.

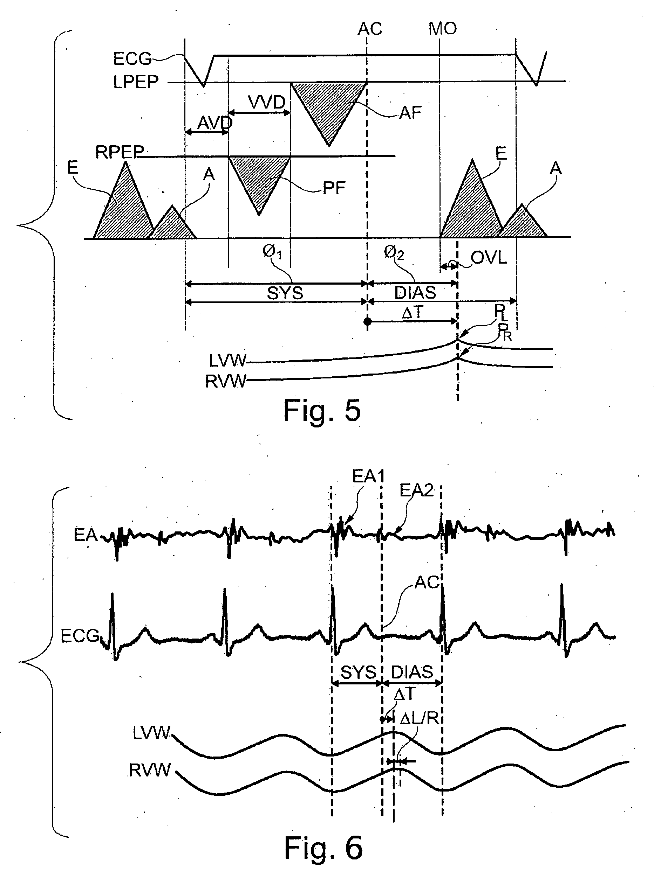

Plus particulièrement, pour la mise en oeuvre de l'invention il s'agit de déterminer le temps T_{stEA2} de début de la composante EA2, qui peut être obtenu par exemple en seuillant une enveloppe d'énergie elle-même obtenue en élevant au carré la valeur des échantillons de signal (enveloppe illustrée en tiretés sur la Figure 4) puis en appliquant une fenêtre de lissage, de 100 millisecondes par exemple. Le temps T_{stEA2} correspondra au franchissement d'un seuil qui peut être par exemple 10 % du maximum de l'énergie sur la fenêtre considérée correspondant à la composante EA2. Sur la Figure 5 on a illustré les différents paramètres d'une pathologie typique de désynchronisation temporelle du ventricule gauche, sous forme d'un diagramme échocardiographique avec les chronogrammes suivants :
- le signal ECG, marquant notamment le début de la contraction auriculaire ;
- la phase de pré-éjection gauche LPEP, montrant notamment le flux aortique AF ;
- la phase de pré-éjection droite RPEP, montrant notamment le flux pulmonaire PF ;
- la phase de post-éjection, avec l'onde E correspondant au remplissage passif, et l'onde A correspondant à la contribution au remplissage par la contraction auriculaire ; et
- les signaux LVW et RVW délivrés respectivement par les capteurs de mouvement des parois du ventricule gauche et du ventricule droit.

La fin de l'écoulement du sang dans l'aorte (fin du flux AF) marque l'achèvement de la phase systolique SYS et, après fermeture de la valve aortique AC, le début de la phase diastolique DIAS.

Les deux dernières traces LVW et RVW présentent des pics respectifs P_{L} et P_{R} qui sont ici concomitants, ce qui signifie que les parois du ventricule gauche et celles du ventricule droit achèvent leur contraction en même temps, et qu'il n'y a donc pas de désynchronisation spatiale.

En revanche, le diagramme révèle une désynchronisation temporelle, dans la mesure où les pics P_{L} et P_{R}, qui marquent la fin de la contraction des ventricules, se situent postérieurement à la fermeture de la valve aortique AC (alors que, idéalement, ces pics devraient être concomitants à cette fermeture), avec un retard ΔT.

Dans l'exemple illustré, on est même en présence d'un phénomène de chevauchement ou *overlap* OVL, dans la mesure où les pics de contraction ventriculaire se situent postérieurement à l'instant MO d'ouverture de la valvule mitrale et viennent donc empiéter sur le remplissage passif E du ventricule, à contretemps avec le comportement hémodynamique normal que l'on devrait avoir.

En d'autres termes, la contraction systolique Ø1 du ventricule gauche, normale, est prolongée par une contraction diastolique Ø 2, pathologique, qu'il convient de diagnostiquer et traiter pour rétablir un fonctionnement convenable du coeur, notamment du point de vue hémodynamique.

La Figure 6 est une autre représentation d'une situation de désynchronisation ventriculaire, montrant :
- l'accélération endocardiaque EA ;
- l'électrocardiogramme ECG ;et
- les signaux LVW et RVW représentatifs des déplacements des parois des ventricules respectivement gauche et droit.

Dans la situation illustrée, on est non seulement en présence d'une désynchronisation temporelle (retard ΔT du pic du signal LVW par rapport à la fermeture de la valve aortique AC, correspondant au début du second pic EA2 du signal EA) mais également d'une désynchronisation spatiale Δ_{L/R}, révélée par le décalage relatif des pics LVW et RVW (en l'espèce, la contraction du ventricule droit étant plus tardive que celle du ventricule gauche).

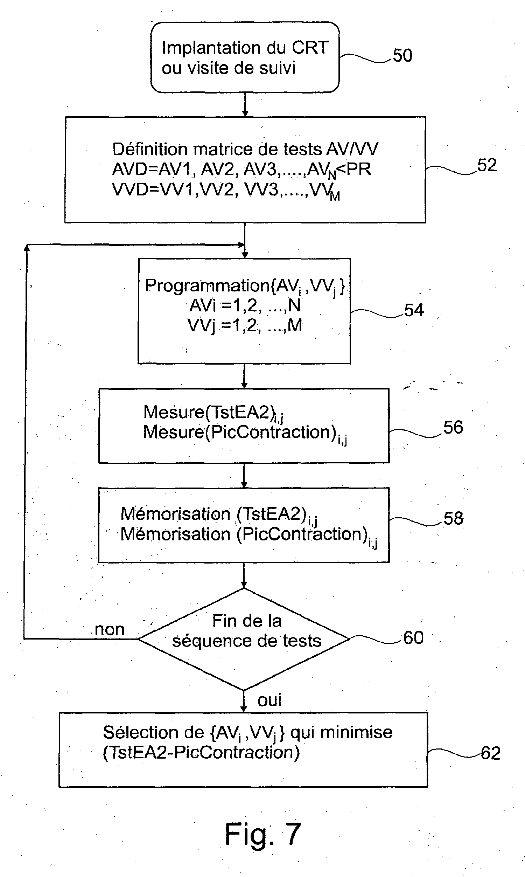

La Figure 7 est un organigramme illustrant les différentes étapes du procédé selon l'invention de diagnostic et de traitement de la désynchronisation ventriculaire temporelle.

Au moment de l'implantation du dispositif ou lors d'une visite ultérieure de suivi chez le médecin (étape 50), le dispositif définit (étape 52) une matrice de test avec différentes combinaisons de délais atrioventriculaires AVD et interventriculaires VVD possibles.

Le dispositif est alors programmé avec chacun de ces couples de délais {AVᵢ, VVⱼ} (étape 54). Puis il détermine (étape 56), pour le couple de valeurs programmé, l'instant T_{stEA2ᵢⱼ} correspondant, représentatif de l'instant de fermeture de la valve aortique AC, ainsi que l'instant de survenue du pic de contraction, représentatif de l'instant auquel tous les segments de la paroi du ventricule gauche ont fini de se contracter.

Les valeurs obtenues sont mémorisées (étape 58) et le dispositif passe au couple de valeurs programmées suivant (étape 60).

Les étapes 54 à 60 sont réitérées jusqu'à ce que tous les couples de valeurs {AVᵢ, VVᵢ} préalablement définies aient été programmés et testés.

Le dispositif sélectionne alors (étape 62) celui de ces couples de valeurs qui minimise (idéalement, qui annule) l'écart temporel entre le marqueur de référence T_{stEA2ᵢⱼ} et le pic de contraction du ventricule gauche.

On considère en effet que la combinaison des DAV et DVV qui procurent la meilleure concordance correspond à la meilleure condition de resynchronisation temporelle. Une différence minimale légèrement positive ou nulle indique que l'on peut s'attendre à ce que tous les segments, ou pratiquement tous les segments, de la paroi du ventricule gauche se soient contractés avant la fermeture de la valve aortique. Une différence minimale négative signifie que, bien que la resynchronisation temporelle ait été améliorée, les segments ventriculaires ne se seront pas tous contractés avant la fermeture de la valve aortique.

Dans le cas d'un dispositif entièrement implanté, ce test d'ajustement des DAV et DVV peut être répété de façon automatique, par exemple hebdomadairement ou mensuellement, pour prendre en compte une modification éventuelle de la situation du patient, notamment du fait des conséquences d'un remodelage ventriculaire, ou pour toute autre raison qui produirait à nouveau une contraction diastolique.

Le diagnostic de désynchronisation temporelle du ventricule selon l'invention peut également être utilisé en combinaison avec des algorithmes d'optimisation automatique des DAV et DVV tels que ceux décrits dans le EP 2 357 020 A1 (Sorin CRM), qui analyse la caractéristique sigmoïde typique de variation du DAV, et/ou dans le EP 1 736 203 A1 (ELA Medical) qui évalue un indice de performance hémodynamique en fonction de l'aire délimitée par cette caractéristique.

Si la différence entre l'optimum donné par l'algorithme et celui donné par l'évaluation selon l'invention (le couple {DAV, DVV} qui minimise l'écart temporel entre le marqueur de référence et le pic de contraction du ventricule gauche) est négative ou nulle, il n'y a pas de désynchronisation temporelle avérée et les valeurs de DAV et DVV calculées par l'algorithme peuvent être retenues comme optimales. Si en revanche cette différence est positive, le test d'autres valeurs de DAV et DVV que celles proposées par l'algorithme peut améliorer la resynchronisation temporelle, avec un impact minimal sur la resynchronisation spatiale.

## Revendications

1. Un dispositif d'évaluation et de thérapie de la désynchronisation ventriculaire temporelle chez un patient, du type comprenant :
- un capteur hémodynamique (32, 36), apte à délivrer un signal (EA) représentatif d'un flux sanguin dans les cavités gauches du myocarde ;
- au moins un capteur de mouvement (40, 44), apte à délivrer un signal (LVW) représentatif d'un déplacement des parois du ventricule gauche du myocarde ;
- des premiers moyens d'analyse, aptes à déterminer un instant (AC) de fermeture de la valve aortique à partir du signal du capteur hémodynamique ;
- des seconds moyens d'analyse, aptes à détecter et évaluer un pic de contraction du ventricule gauche à partir du (des) signal(ux) du(des) capteur(s) de mouvement ; et
- des moyens de stimulation bi-, tri- ou multiventriculaire, aptes à délivrer des impulsions de stimulation destinées à être appliquées à des électrodes implantées respectivement en au moins un site de stimulation ventriculaire droit et au moins un site de stimulation ventriculaire gauche selon une configuration de stimulation courante avec application d'au moins un délai interventriculaire DVV et/ou atrioventriculaire DAV modifiables,
dispositif **caractérisé en ce que** :
- les seconds moyens d'analyse sont aptes à déterminer un instant (P_{L}) dudit pic de contraction du ventricule gauche ;
et **en ce qu'**il comprend en outre :
- des troisièmes moyens d'analyse, aptes à mesurer un écart temporel (ΔT) entre l'instant (P_{L}) du pic de contraction du ventricule gauche et l'instant (AC) de fermeture de la valve aortique ; et
- des moyens aptes, si les troisièmes moyens d'analyse déterminent que l'instant (P_{L}) du pic de contraction du ventricule gauche est postérieur à l'instant (AC) de fermeture de la valve aortique, à modifier le(s) DVV et/ou DAV de la configuration de stimulation courante dans un sens réduisant et annulant le retard (ΔT) du pic de contraction du ventricule gauche après fermeture de la valve aortique.

2. Le dispositif de la revendication 1, dans lequel le capteur hémodynamique est un capteur du groupe comprenant : capteur implantable ou externe d'accélération endocardiaque (32, 36) ; capteur implantable de bioimpédance ; capteur implantable ou externe de détection de l'onde T ; capteur implantable de pression ventriculaire ; et les combinaisons de tels capteurs.

3. Le dispositif de la revendication 1, dans lequel le au moins un capteur de mouvement est un capteur du groupe comprenant : capteur de mouvement implantable en un site endocavitaire, épicardique (44) ou endocoronarien (40) ; capteur externe de mouvement ; capteur de recueil d'un signal de tomographie de champ ; et les combinaisons de tels capteurs.

4. Le dispositif de la revendication 1, dans lequel :
- le capteur hémodynamique est un capteur implantable (32, 36) apte à délivrer un signal d'accélération endocardiaque EA ; et
- les premiers moyens d'analyse sont des moyens aptes à :
· isoler dans le signal EA une composante EA2 correspondant au second pic d'accélération endocardiaque associé à la relaxation ventriculaire isovolumique sur un cycle cardiaque compris entre deux évènements ventriculaires successifs, et
· déterminer comme instant (AC) de fermeture de la valve aortique l'instant (t_{stEA2}) d'apparition du début de la composante EA2.

5. Le dispositif de la revendication 4, dans lequel les premiers moyens d'analyse sont des moyens aptes à déterminer comme instant (t_{stEA2}) d'apparition du début de la composante EA2 un instant de franchissement d'un seuil d'enveloppe d'énergie de la composante EA2.

6. Le dispositif de la revendication 1, dans lequel :
- il est prévu au moins deux capteurs de mouvement (40, 44 ; 48), aptes à délivrer des signaux (LVW ; RVW) représentatifs des déplacements respectifs des parois du ventricule gauche et droit du myocarde ;
- les seconds moyens d'analyse sont aptes à déterminer les instants des pics de contraction (P_{L} ; P_{R}) respectifs des ventricules gauche et droit ; et
- il est en outre prévu des moyens d'évaluation de la désynchronisation ventriculaire spatiale chez le patient, aptes à déterminer un défaut de concomitance (Δ_{L/R}) des pics de contraction respectifs des ventricules gauche et droit.

7. Le dispositif de la revendication 6, comprenant en outre :
- des moyens de stimulation bi-, tri- ou multiventriculaire, aptes à délivrer des impulsions de stimulation destinées à être appliquées à des électrodes implantées respectivement en au moins un site de stimulation ventriculaire droit et au moins un site de stimulation ventriculaire gauche selon une configuration de stimulation courante avec application d'au moins un délai interventriculaire DVV et/ou atrioventriculaire DAV modifiables ;
- des moyens aptes, si les moyens d'évaluation de la désynchronisation temporelle déterminent que les instants des pics de contraction (P_{L} ; P_{R}) des ventricules gauche et droit sont tous deux postérieurs à l'instant (AC) de fermeture de la valve aortique, à modifier le(s) DVV et/ou DAV de la configuration de stimulation courante dans un sens qui i) prioritairement, réduite et annule le retard (Δ_{T}) de ces deux pics de contraction après fermeture de la valve aortique et ii) subsidiairement, réduit l'écart temporel (Δ_{L/R}) entre ces deux pics de contraction.

## Patentansprüche

1. Vorrichtung zur Bewertung und Therapie der zeitlichen ventrikulären Desynchronisation bei einem Patienten, des Typs, welcher aufweist:
- einen hämodynamischen Sensor (32, 36), der geeignet ist, ein Signal (EA) zu liefern, das für einen Blutfluss in den linken Kavitäten des Myokards repräsentativ ist;
- mindestens einen Bewegungssensor (40, 44), der geeignet ist, ein Signal (LVW) zu liefern, das für eine Verschiebung der Wände des linken Ventrikels des Myokards repräsentativ ist;
- erste Analysemittel, die geeignet sind, einen Zeitpunkt (AC) des Schließens der Aortenklappe ausgehend von dem Signal des hämodynamischen Sensors zu bestimmen;
- zweite Analysemittel, die geeignet sind, eine Kontraktionsspitze des linken Ventrikels ausgehend von dem (den) Signal(en) des Bewegungssensors (der Bewegungssensoren) zu erfassen und zu bewerten; und
- Mittel zur bi-, tri- oder multiventrikulären Stimulation, die geeignet sind, Stimulationsimpulse zu liefern, die dazu bestimmt sind, an Elektroden angelegt zu werden, die an mindestens einem Ort der rechtsventrikulären Stimulation bzw. mindestens einem Ort der linksventrikulären Stimulation gemäß einer aktuellen Stimulationskonfiguration implantiert sind, mit Anwendung mindestens einer modifizierbaren interventrikulären DVV und/oder atrioventrikulären DAV Verzögerung,
wobei die Vorrichtung **dadurch gekennzeichnet ist, dass**:
- die zweiten Analysemittel geeignet sind, einen Zeitpunkt (P_{L}) der Kontraktionsspitze des linken Ventrikels zu bestimmen;
und dadurch, dass sie außerdem aufweist:
- dritte Analysemittel, die geeignet sind, einen zeitlichen Abstand (ΔT) zwischen dem Zeitpunkt (P_{L}) der Kontraktionsspitze des linken Ventrikels und dem Zeitpunkt (AC) des Schließens der Aortenklappe zu messen; und
- Mittel, die geeignet sind, falls die dritten Analysemittel bestimmen, dass der Zeitpunkt (P_{L}) der Kontraktionsspitze des linken Ventrikels später als der Zeitpunkt (AC) des Schließens der Aortenklappe ist, die DVV und/oder DAV der aktuellen Stimulationskonfiguration in einer Richtung zu modifizieren, welche die Verzögerung (ΔT) der Kontraktionsspitze des linken Ventrikels nach dem Schließen der Aortenklappe reduziert und beseitigt.

2. Vorrichtung nach Anspruch 1, wobei der hämodynamische Sensor ein Sensor aus der Gruppe ist, welche umfasst: einen implantierbaren oder externen Sensor für die endokardiale Beschleunigung (32, 36); einen implantierbaren Bioimpedanzsensor; einen implantierbaren oder externen Sensor zur Erfassung der T-Welle; einen implantierbaren Sensor für den ventrikulären Druck; und die Kombinationen solcher Sensoren.

3. Vorrichtung nach Anspruch 1, wobei der mindestens eine Bewegungssensor ein Sensor aus der Gruppe ist, welche umfasst: einen Bewegungssensor, der an einem endokavitären, epikardialen (44) oder endokoronaren (40) Ort implantierbar ist; einen externen Bewegungssensor; einen Sensor zum Empfang eines Feldtomographie-Signals; und die Kombinationen solcher Sensoren.

4. Vorrichtung nach Anspruch 1, wobei:
- der hämodynamische Sensor ein implantierbarer Sensor (32, 36) ist, der geeignet ist, ein endokardiales Beschleunigungssignal EA zu liefern; und
- die ersten Analysemittel Mittel sind, die geeignet sind:
. in dem Signal EA eine Komponente EA2 zu isolieren, die der zweiten Spitze der endokardialen Beschleunigung entspricht, die der isovolumischen ventrikulären Relaxation in einem Herzzyklus zwischen zwei aufeinander folgenden ventrikulären Ereignissen zugeordnet ist, und
. als Zeitpunkt (AC) des Schließens der Aortenklappe den Zeitpunkt (t_{stEA2}) des Erscheinens des Anfangs der Komponente EA2 zu bestimmen.

5. Vorrichtung nach Anspruch 4, wobei die ersten Analysemittel Mittel sind, die geeignet sind, als Zeitpunkt (t_{stEA2}) des Erscheinens des Anfangs der Komponente EA2 einen Zeitpunkt der Überschreitung eines Schwellenwertes einer Energieeinhüllenden der Komponente EA2 zu bestimmen.

6. Vorrichtung nach Anspruch 1, wobei:
- mindestens zwei Bewegungssensoren (40, 44; 48) vorgesehen sind, die geeignet sind, Signale (LVW; RVW) zu liefern, die für die Verschiebungen der Wände des linken bzw. des rechten Ventrikels des Myokards repräsentativ sind;
- die zweiten Analysemittel geeignet sind, die Zeitpunkte der Kontraktionsspitzen (P_{L}; P_{R}) des linken bzw. des rechten Ventrikels zu bestimmen; und
- außerdem Mittel zur Bewertung der räumlichen ventrikulären Desynchronisation bei dem Patienten vorgesehen sind, die geeignet sind, einen Gleichzeitigkeitsfehler (Δ_{L/R}) der jeweiligen Kontraktionsspitzen des linken und rechten Ventrikels zu bestimmen.

7. Vorrichtung nach Anspruch 6, welche außerdem aufweist:
- Mittel zur bi-, tri- oder multiventrikulären Stimulation, die geeignet sind, Stimulationsimpulse zu liefern, die dazu bestimmt sind, an Elektroden angelegt zu werden, die an mindestens einem Ort der rechtsventrikulären Stimulation bzw. mindestens einem Ort der linksventrikulären Stimulation gemäß einer aktuellen Stimulationskonfiguration implantiert sind, mit Anwendung mindestens einer modifizierbaren interventrikulären DVV und/oder atrioventrikulären DAV Verzögerung;
- Mittel, die geeignet sind, falls die Mittel zur Bewertung der zeitlichen Desynchronisation bestimmen, dass die Zeitpunkte der Kontraktionsspitzen (P_{L}; P_{R}) des linken und rechten Ventrikels alle beide nach dem Zeitpunkt (AC) des Schließens der Aortenklappe liegen, die DVV und/oder DAV der aktuellen Stimulationskonfiguration in einer Richtung zu modifizieren, welche i) vorrangig die Verzögerung (ΔT) dieser zwei Kontraktionsspitzen nach dem Schließen der Aortenklappe reduziert und beseitigt, und ii) hilfsweise den zeitlichen Abstand (Δ_{L/R}) zwischen diesen zwei Kontraktionsspitzen verkleinert.

## Claims

1. A device for assessment and therapy of temporal ventricular desynchronization in a patient, of the type comprising:
- an hemodynamic sensor (32, 36), adapted to deliver a signal (EA) representative of a flow of blood in the left cavities of the myocardium;
- at least one movement sensor (40, 44), adapted to deliver a signal (LVW) representative of a displacement of the walls of the left ventricle of the myocardium;
- first analysis means, adapted to determine an instant (AC) of closure of the aortic valve based on the signal of the hemodynamic sensor;
- second analysis means, adapted to detect and assess a peak of contraction of the left ventricle based on the signal(s) of the movement sensor(s); and
- means for bi-, tri- or multi-ventricular stimulation, adapted to deliver stimulation pulses intended to be applied to electrodes respectively implanted in at least one right ventricular stimulation site and at least one left ventricular stimulation site, according to a current stimulation configuration with application of at least one modifiable interventricular delay VVD and/or atrioventricular delay AVD,
the device being **characterized in that**:
- the second analysis means are adapted to determine an instant (P_{L}) of said peak of contraction of the left ventricle;
and **in that** it further comprises:
- third analysis means, adapted to measure a time difference (ΔT) between the instant (P_{L}) of the peak of contraction of the left ventricle and the instant (AC) of closure of the aortic valve; and
- means adapted, if the third analysis means determine that the instant (P_{L}) of the peak of contraction of the left ventricle is posterior to the instant (AC) of closure of the aortic valve, to modify the VVD and/or AVD of the current stimulation configuration in a direction reducing and cancelling the delay (ΔT) of the peak of contraction of the left ventricle after the closure of the aortic valve.

2. The device of claim 1, wherein the hemodynamic sensor is a sensor of the group comprising: implantable or external endocardial acceleration sensor (32, 36); implantable bioimpedance sensor; implantable or external T-wave detection sensor; implantable ventricular pressure sensor; and the combinations of such sensors.

3. The device of claim 1, wherein the at least one movement sensor is a sensor of the group comprising: movement sensor implantable in an endocavitary, epicardial (44) or endocoronary (40) sensor; external movement sensor; sensor for collecting a field tomography signal; and the combinations of such sensors.

4. The device of claim 1, wherein:
- the hemodynamic sensor is an implantable sensor (32, 36) adapted to deliver an endocardial acceleration EA signal; and
- the first analysis means are means adapted to:
. isolate in the EA signal a component EA2 corresponding to the second peak of endocardial acceleration associated with the isovolumic ventricular relaxation over a cardiac cycle comprised between two successive ventricular events, and
. determine as the instant (AC) of closure of the aortic valve the instant (t_{stEA2}) of appearance of the beginning of the component EA2.

5. The device of claim 4, wherein the first analysis means are means adapted to determine as the instant (t_{stEA2}) of appearance of the beginning of the component EA2 an instant of crossing of a threshold of energy envelop of the component EA2.

6. The device of claim 1, wherein:
- at least two movement sensors (40, 44; 48) are provided, which are adapted to deliver signals (LVW; RVW) representative of the respective displacements of the walls of the left and right ventricles of the myocardium;
- the second analysis means are adapted to determine the instants of the respective peaks of contraction (P_{L}; P_{R}) of the left and right ventricles; and
- means are further provided for assessing the spatial ventricular desynchronization in the patient, adapted to determine a failure of concomitance (Δ_{L/R}) of the respective peaks of contraction of the left and right ventricles.

7. The device of claim 6, further comprising:
- means for bi-, tri- or multi-ventricular stimulation, adapted to deliver stimulation pulses intended to be applied to electrodes respectively implanted in at least one right ventricular stimulation site and at least one left ventricular stimulation site, according to a current stimulation configuration with application of at least one modifiable interventricular delay VVD and/or atrioventricular delay AVD,
- means adapted, if the temporal desynchronization assessment means determine that the instants of the peaks of contraction (P_{L}; P_{R}) of the left and right ventricles are both posterior to the instant (AC) of closure of the aortic valve, to modify the VVD and/or AVD of the current stimulation configuration in a direction that i) as a priority, reduces and cancels the time-lag (ΔT) of these two peaks of contraction after the closure of the aortic valve and ii) subsidiarily, reduces the time difference (Δ_{L/R}) between these two peaks of contraction.
